# EUROPÄISCHE PATENTSCHRIFT

(11) **EP 1 953 546 B1**
(45) Veröffentlichungstag und Bekanntmachung des Hinweises auf die Patenterteilung: **24.03.2010**
(21) Anmeldenummer: 07002376.7
(22) Anmeldetag: 03.02.2007
(51) Int. Cl.: G01N 33/487

(54) **Diagnostische Testbandeinheit, insbesondere Testbandkassette**
Diagnostic test tape unit, in particular test tape cassette
Unité de bande de test diagnostique, en particulier une cassette à bande de test

(43) Veröffentlichungstag der Anmeldung: 06.08.2008
(73) Patentinhaber: F.HOFFMANN-LA ROCHE AG, 4070 Basel (CH); Roche Diagnostics GmbH, 68305 Mannheim (DE)
(72) Erfinder: Sacherer, Klaus Dieter, 67281 Kirchheim (DE)
(74) Vertreter: Pfiz, Thomas

(56) Entgegenhaltungen:
- EP-A1- 1 267 168
- EP-A1- 1 360 935
- EP-A1- 1 475 330
- EP-A2- 0 299 517
- WO-A-2004/047642
- JP-A- 2001 315 493

## Beschreibung

Die Erfindung betrifft eine diagnostische Bandeinheit, insbesondere Bandkassette mit einem diagnostische Testelemente und/oder Stechelemente aufweisenden Band, das von einer ersten Spule abwickelbar und auf eine zweite Spule aufwickelbar ist, wobei ein zwischen den Spulen befindlicher freier Bandabschnitt für einen Benutzer in Gebrauch bringbar ist.

In einer früheren Anmeldung WO2004/047642 der Anmelder ist ein Testgerät mit einer Bandeinheit dieser Art zum Durchführen von Blutglucosetests bekannt. Dort wird unter anderem eine seitlich nebeneinander liegende, koaxiale Spulenanordnung vorgeschlagen, um den begrenzten Bauraum in einem Handgerät möglichst effizient auszunutzen. Hierbei wird allerdings die Bauhöhe des Geräts mindestens von der doppelten Bandbreite und zusätzlichen Zwischenwanddicken bestimmt.

Kompakt aufgebaute Bandeinheiten sind bereits bekannt, diese betreffen aber andere technische Gebiete wie Korrekturband oder Klebeband, siehe z.B. JP 2001 315 493 und EP 1 475 330.

Ausgehend hiervon liegt der Erfindung die Aufgabe zugrunde, die im Stand der Technik bekannten Systeme weiter zu entwickeln und eine Bandeinheit der eingangs angegebenen Art im Sinne eines kompakten Aufbaus insbesondere für ein handliches Gerätesystem zu optimieren.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die Spulen flach ineinander anzuordnen. Dementsprechend wird erfindungsgemäß vorgeschlagen, dass eine der Spulen einen zentralen Aufnahmebereich umschließt, und dass die andere Spule in dem Aufnahmebereich angeordnet ist. Auf diese Weise kann eine weitere Miniaturisierung und insbesondere eine platzsparende flache Bauform erreicht werden. Dadurch ist es auch möglich, die Abstände zwischen den auf dem Band transportierten Elementen und damit letztlich auch die Gesamtlänge des Bands zu verringern.

Für eine möglichst geringe Bauhöhe ist es von Vorteil, wenn dass die beiden Spulen auf gleicher Höhe ineinander liegen, wobei die Stirnseiten der äußeren Spule den Aufnahmebereich axial begrenzen.

Um die Handhabung für einen Benutzer zu erleichtern, ist es vorteilhaft, wenn die beiden Spulen in einem Gehäuse gelagert sind und die so gebildete Bandkassette als Verbrauchsartikel für ein diagnostisches Gerät ausgebildet ist. Eine weitere Verbesserung kann dadurch erzielt werden, dass mindestens eine der Spulen einen motorisch oder manuell drehbaren Wickelkern zur Aufnahme eines Bandwickels aufweist.

Um Bandumlenkungen zu vereinfachen, ist es vorteilhaft, wenn die innere Spule in dem Aufnahmebereich exzentrisch oder konzentrisch zu der umgebenden äußeren Spule angeordnet ist, so dass die Spulenachsen in einem Abstand parallel zueinander oder aufeinander liegen.

Für den Einsatz in einem diagnostischen Gerät ist es von Vorteil, wenn in dem Aufnahmebereich vorzugsweise innerhalb der darin befindlichen Spule eine Messkammer für eine das Band zweckmäßig optisch abtastende Messeinheit freigehalten ist.

Eine weitere vorteilhafte Ausgestaltung der Erfindung sieht vor, dass der freie Bandabschnitt über eine Umlenkführung stirnseitig über die äußere Spule übergeführt ist, um den Bandtransport zwischen der äußeren und inneren Spule zu ermöglichen. In bevorzugter Ausführung ist der freie Bandabschnitt über ein außerhalb des Aufnahmebereichs angeordnetes erstes Umlenkelement und ein innerhalb des Aufnahmebereichs angeordnetes zweites Umlenkelement geführt.

Um eine Bandlängsdrehung zu ermöglichen, ist es von Vorteil, die Umlenkelemente durch vorzugsweise kegelstumpfförmige Umlenkrollen gebildet sind. Eine Richtungsänderung des Bandtransports lässt sich vorteilhafterweise auch dadurch bewerkstelligen, dass das Band an mindestens einer Umlenkstelle über zwei unter voneinander verschiedene Richtungen verlaufende, vorzugsweise gerundete Umlenkkanten geführt ist.

Eine weitere Verbesserung sieht vor, dass die Umlenkführung eine an einer Stirnseite der Spulen angeordnete, vorzugsweise durch eine Gleitfläche oder eine Dichtung gebildete Überleitstruktur für das Band aufweist.

Im Sinne einer möglichst flachen Bauform ist es weiterhin vorteilhaft, wenn der freie Bandabschnitt unter Längsverwindung stirnseitig über die äußere Spule übergeleitet ist, so dass der Bandabschnitt in einem Überleitbereich parallel zur Stirnseite der Spule verläuft.

Ein bevorzugter Einsatzzweck im diagnostischen Bereich liegt darin, dass das Band abschnittsweise mit analytischen Testfeldern versehen ist, und dass die Testfelder an einer Applikationsstelle sukzessive mit einer Testflüssigkeit, insbesondere Körperflüssigkeit wie Blut beaufschlagbar sind.

Alternativ oder ergänzend ist es auch möglich, dass mittels des Bands transportierte Stechelemente an einer Applikationsstelle an dem freien Bandabschnitt bereitstellbar sind. Damit kann ein Magazinsystem für eine Vielzahl von Blutzuckertests realisiert werden.

Für möglichst lange Haltbarkeit ist es günstig, wenn die durch ungebrauchtes Band gebildete erste Spule zweckmäßig in dem Aufnahmebereich gegenüber der Umgebung vor allem gegen Feuchtezutritt abgeschirmt ist.

Gegenstand der Erfindung ist auch ein diagnostisches System mit einer vorstehend beschriebenen Bandeinheit, wobei zumindest eine Spule der Bandeinheit motorisch oder manuell drehbar ist, um auf dem Band magazinierte Elemente nacheinander in Einsatz zu bringen.

Im Folgenden wird die Erfindung anhand der in der Zeichnung schematisch dargestellten Ausführungsbeispiele näher erläutert. Es zeigen:
- Fig. 1: eine diagnostische Bandkassette mit ineinander angeordneten Spulen in der Draufsicht;
- Fig. 2: die Bandkassette nach Fig. 1 in einer Seitenansicht;
- Fig. 3: die vorgespulte Bandkassette in Fig. 1 entsprechender Darstellung;
- Fig. 4: eine weitere Ausführungsform einer kompakten Bandkassette in axial geschnittener perspektivischer Darstellung;
- Fig. 5: eine Explosionsdarstellung der Bauteile der Kassette nach Fig. 4;
- Fig. 6 und 7: noch eine weitere Ausführungsform einer Bandkassette mit ineinander angeordneten Spulen in der Draufsicht und Perspektive.

Die in der Zeichnung dargestellte diagnostische Bandkassette 10 umfasst ein flexibles Testband 12, eine Abwickelspule 14 zum Abwickeln von ungebrauchtem Testband und eine Aufwickelspule 16 zum Aufwickeln von gebrauchtem Testband, wobei der momentan zwischen den Spulen 14, 16 befindliche freie Bandabschnitt 18 an einer Applikationsstelle (Pfeil 20) in Gebrauch bringbar ist. Dies kann dadurch erfolgen, dass abschnittsweise auf dem Testband 12 befindliche analytische Testfelder 22 mit einer Testflüssigkeit, z.B. Blut beaufschlagbar sind, um einen Analyten (Glucose) nachzuweisen. Zweckmäßig lässt sich eine solche Bandkassette 10 in einem nicht gezeigten Handgerät als Testsystem einsetzen, wobei die Testfelder 22 durch einen mit der Aufwickelspule 16 koppelbaren Drehantrieb durch Bandvorspulen sukzessive bereitstellbar sind.

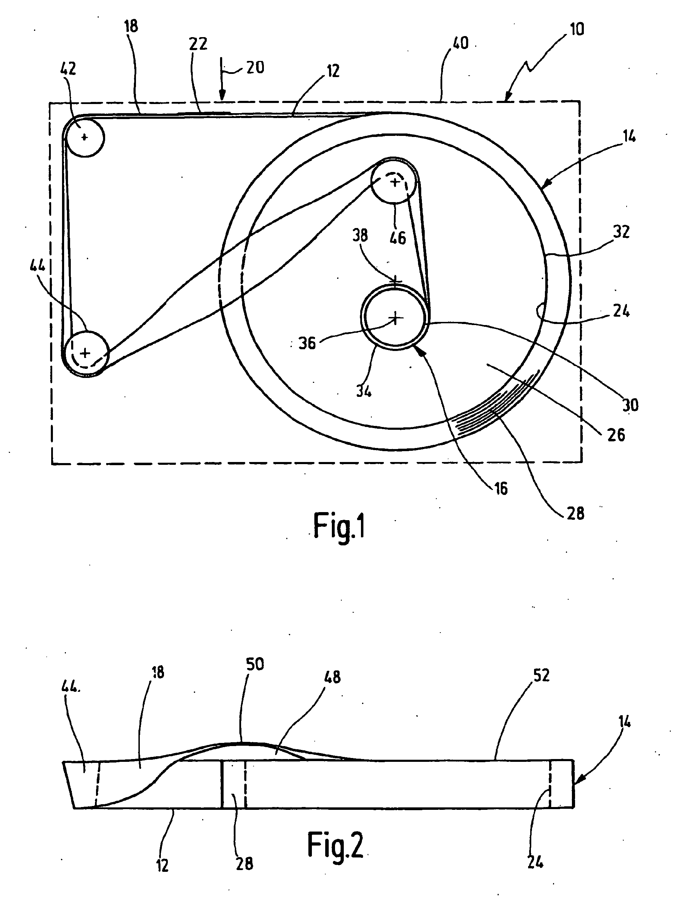

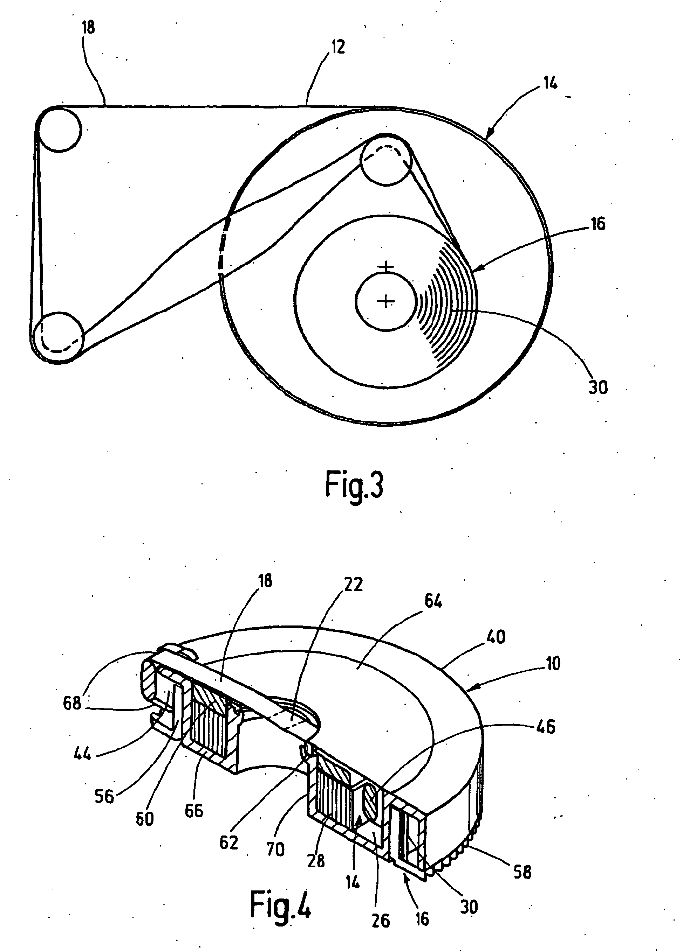

Wie aus Fig. 1 und 3 ersichtlich, umschließt die Abwickelspule 14 mit einer inneren Ringfläche 24 einen zentralen Aufnahmebereich 26, in welchem die Aufwickelspule 16 angeordnet ist, um eine kompakte Bauform zu erzielen. Die Spulen 14, 16 bzw. die aus dem Testband 12 gebildeten Bandwickel 28, 30 liegen somit auf gleicher Ebene bzw. Höhe ineinander. Hierbei ist in der gezeigten Ausführungsform der Trägerdurchmesser 32 der Abwickelspule 14 um ein Mehrfaches größer als der Trägerdurchmesser 34 der Aufwickelspule 16, so dass für den durch Vorspulen größer werdenden Bandwickel 30 ein genügend größer Aufnahmebereich 26 zur Verfügung steht (Fig. 3). Zweckmäßig ist die Aufwickelspule 16 exzentrisch in der Abwickelspule 14 angeordnet, wobei die betreffenden Spulenachsen 36, 38 im Abstand parallel zueinander verlaufen.

Der freie Bandabschnitt 18 ist innerhalb des Kassettengehäuses 40 über drei Umlenkrollen 42, 44, 46 geführt. Die beiden Umlenkrollen 44, 46 sorgen zusammen mit dem aus Fig. 2 ersichtlichen Überleitstück 48 als Umlenkführung dafür, dass der freie Bandabschnitt 18 flach über den äußeren Bandwickel 28 hinweg geführt wird. Zu diesem Zweck sind die Umlenkrollen 44, 46 kegelförmig ausgebildet, so dass im Scheitelpunkt der Gleitfläche 50 des Überleitstücks 48 eine Bandverdrehung bzw. Längsverwindung des Testbands 12 um 90° erreicht wird. Das Band verläuft somit im oberen Verwindungsbereich senrecht zu den Spulenachsen 36, 38 und parallel zur überleitseitigen Stirnfläche 52 der Spule 14. Denkbar ist es auch, dass in diesem Bereich eine Gehäuseöffnung als Applikationsstelle 20 vorhanden ist. Anstelle von Testfeldern 22 kann das Testband 12 auch nicht gezeigte Stechelemente aufweisen, um ein Abspenden an der Applikationsstelle zu ermöglichen. Möglich ist es auch, dass auf dem Band Stechelemente und Testelemente kombiniert angeordnet sind, um die Abläufe für einen Blutzuckertest (Stechen, Messen) weiter zu vereinfachen.

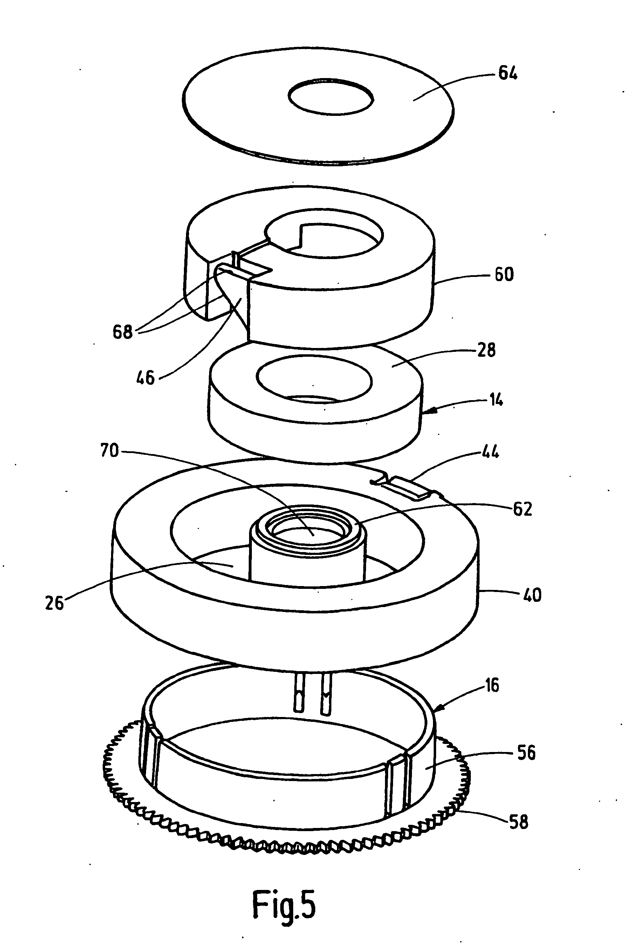

Bei den folgenden Ausführungsbeispielen sind entsprechende Teile mit gleichen Bezugszeichen versehen, wie vorstehend beschrieben. Fig. 4 zeigt eine besonders kompakte Bauform ähnlich einem Donut. Die einzelnen Bauteile sind am besten aus Fig. 5 ersichtlich. Die Spulen 14, 16 sind hier in einem ringförmigen Gehäuse 40 konzentrisch zueinander angeordnet. In diesem Fall bildet die nur aus einem Bandwickel 28 bestehende innere Spule 14 den ungebrauchten Teil, während die äußere Spule 16 für das Aufwickeln von gebrauchtem Testband vorgesehen ist. Zu diesem Zweck weist die Aufwickelspule 16 einen Wickelkern 56 auf, der über einen stirnseitigen Zahnring 58 drehbar ist. Hierbei wird das Testband 12 als freier Bandabschnitt 18 radial übergeleitet.

Die Umlenkführung umfasst ein an dem Gehäuse 40 ausgebildetes erstes Umlenkstück 44 und ein an einem aus Trockenmittel geformten Gehäuseeinsatz 60 angeformtes zweites Umlenkstück 46 sowie einen Dichtring 62. Ein Deckel 64 sorgt für eine gegen Feuchtezutritt dichte Abdeckung des Aufnahmebereichs 26 gegenüber dem Gehäuseboden 66. Auch hier definieren die durch die Stirnflächen des äußeren Bandwickels 30 aufgespannten Ebenen die axiale Begrenzung des Aufnahmebereichs 26, so dass die Bandwickel 28, 30 wiederum auf gleicher Höhe ineinander liegen. Um die stirnseitige Bandumlenkung zu ermögichen, besitzen die Umlenkstücke 44, 46 jeweils zwei spitzwinklig zueinander verlaufende gerundete Umlenkkanten 68, über welche das Testband 12 jeweils um 90° aus seiner momentanen Transportrichtung umlenkbar ist. Auf diese Weise kann das Testband 12 tangential zu den Bandwickeln 28, 30 abgezogen bzw. aufgespult werden, während die Überleitung über die äußere Spule 16 in deren radialer Richtung erfolgt.

Um auf dem Testband 12 vorhandene Testfelder 22 optisch abtasten bzw. erfassen zu können, ist im Inneren des Gehäuses 40 eine Messkammer 70 freigehalten, in welche eine nicht gezeigte geräteseitige Messeinheit beim Einsatz der Kassette 10 eingreift.

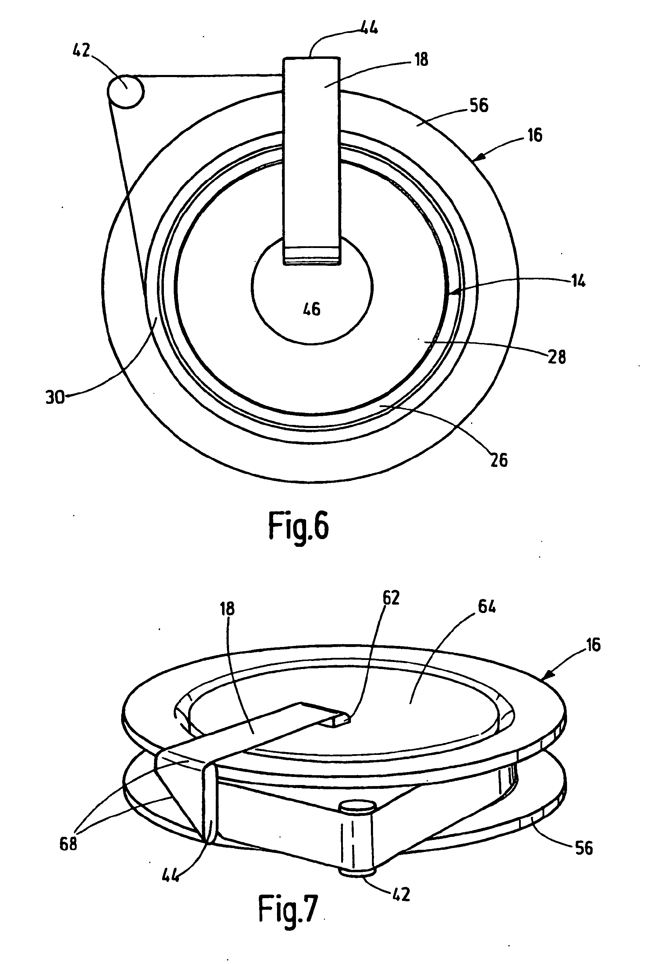

Fig. 6 und 7 zeigen ein ähnliches Prinzip der Band-in-Band-Anordnung, wobei hier das ungebrauchte Testband 12 aus dem.Inneren des Bandwickels 28 der inneren Spule 14 abgezogen wird. Für die Bandumlenkung sind ebenfalls zwei Umlenkstücke 44, 46 mit gegeneinander abgewinkelten Umlenkkanten 68 vorgesehen. Der Aufnahmebereich 26 ist stirnseitig durch einen nur in Fig. 7 gezeigten Deckel 64 verschlossen, durch welchen das Band 12 über eine Dichtung 62 hindurchgeführt ist.

## Patentansprüche

1. Diagnostische Bandeinheit, insbesondere Bandkassette, mit einem diagnostische Testelemente (22) und/oder Stechelemente aufweisenden Band (12), das von einer ersten Spule (14) abwickelbar und auf eine zweite Spule (16) aufwickelbar ist, wobei ein zwischen den Spulen (14,16) befindlicher freier Bandabschnitt (18) für einen Benutzer in Gebrauch bringbar ist, **dadurch gekennzeichnet, dass** eine der Spulen (14, 16) einen zentralen Aufnahmebereich (26) umschließt, und dass die andere Spule (14, 16) in dem Aufnahmebereich (26) angeordnet ist.

2. Bandeinheit nach Anspruch 1, **dadurch gekennzeichnet, dass** die beiden Spulen (14,16) auf gleicher Höhe ineinander liegen, wobei die Stirnseiten der äußeren Spule den Aufnahmebereich (26) axial begrenzen.

3. Bandeinheit nach Anspruch 1 oder 2, **dadurch gekennzeichnet, dass** die beiden Spulen (14,16) in einem Gehäuse gelagert sind, und dass die so gebildete Bandkassette als Verbrauchsartikel für ein diagnostisches Gerät ausgebildet ist.

4. Bandeinheit nach einem der Ansprüche 1 bis 3, **dadurch gekennzeichnet, dass** mindestens eine der Spulen (14,16) einen motorisch oder manuell drehbaren Wickelkern zur Aufnahme eines Bandwickels aufweist.

5. Bandeinheit nach einem der Ansprüche 1 bis 4, **dadurch gekennzeichnet, dass** die in dem Aufnahmebereich (26) befindliche innere Spule (14, 16) exzentrisch oder konzentrisch zu der umgebenden äußeren Spule (14, 16) angeordnet ist.

6. Bandeinheit nach einem der Ansprüche 1 bis 5, **dadurch gekennzeichnet, dass** in dem Aufnahmebereich vorzugsweise innerhalb der darin befindlichen Spule (16) eine Messkammer für eine das Band abtastende Messeinheit freigehalten ist.

7. Bandeinheit nach einem der Ansprüche 1 bis 6, **dadurch gekennzeichnet, dass** der freie Bandabschnitt (18) über eine Umlenkführung (44,46,48) stirnseitig über die äußere Spule (14) übergeführt ist.

8. Handeinheit nach einem der Ansprüche 1 bis 7, **dadurch gekennzeichnet, dass** der freie Bandabschnitt (18) über ein außerhalb des Aufnahmebereichs (26) angeordnetes erstes Umlenkelement (44) und ein innerhalb des Aufnahmebereichs (26) angeordnetes zweites Umlenkelement (46) geführt ist.

9. Bandeinheit nach Anspruch 8, **dadurch gekennzeichnet, dass** die Umlenkelemente durch vorzugsweise kegelstumpfförmige Umlenkrollen (44,46) gebildet sind.

10. Bandeinheit nach einem der Ansprüche 1 bis 9, **dadurch gekennzeichnet, dass** das Band an mindestens einer Umlenkstelle über zwei unter voneinander verschiedene Richtungen verlaufende, vorzugsweise gerundete Umlenkkanten geführt ist.

11. Bandeinheit nach einem der Ansprüche 1 bis 10, **gekennzeichnet durch** eine an einer Stirnseite (52) der spulen (14, 16) angeordnete, vorzugsweise **durch** eine Gleitfläche (50) oder eine Dichtung gebildete Überleitstruktur (48) für das Band (12).

12. Bandeinheit nach einem der Ansprüche 1 bis 11, **dadurch gekennzeichnet, dass** der freie Bandabschnitt (18) unter Längsverwindung stirnseitig über die äußere Spule (14) übergeleitet ist, so dass der Bandabschnitt (18) in einem Überleitbereich parallel zur Stirnseite (52) der Spule (14) verläuft.

13. Bandeinheit nach einem der Ansprüche 1 bis 12, **dadurch gekennzeichnet, dass** das Band (12) abschnittsweise mit analytischen Testfeldern (22) versehen ist, und dass die Testfelder (22) an einer Applikationsstelle (20) sukzessive mit einer Testflüssigkeit, insbesondere Körperflüssigkeit beaufschlagbar sind.

14. Bandeinheit nach einem der Ansprüche 1 bis 11, **dadurch gekennzeichnet, dass** mittels des Bands (12) transportierte Stechelemente an einer Applikationsstelle (20) an dem freien Bandabschnitt (18) bereitstellbar sind.

15. Bandeinheit nach einem der Ansprüche 1 bis 12, **dadurch gekennzeichnet, dass** mindestens die durch ungebrauchtes Band gebildete erste Spule (14) zweckmäßig in dem Aufnahmebereich (26) gegenüber der Umgerbung abgeschirmt ist.

16. Diagnostisches System mit einer Bandeinheit nach einem der vorhergehenden Ansprüche, wobei zumindest eine Spule (14) oder Bandeinheit (10) motorisch oder manuell drehbar ist.

## Claims

1. Diagnostic tape unit and in particular tape cassette with a tape (12) having diagnostic test elements (22) and/or lancing elements which can be unwound from a first spool (14) and wound onto a second spool (16) wherein a free section of tape (18) located between the spools (14, 16) can be brought into use for a user, **characterized in that** one of the spools (14, 16) encloses a central receiving area (26) and that the other spool (14, 16) is arranged in the receiving area (26).

2. Tape unit according to claim 1, **characterized in that** the two spools (14, 16) lie within each other at the same level wherein the front sides of the outer spool axially delimit the receiving area (26).

3. Tape unit according to claim 1 or 2, **characterized in that** the two spools (14, 16) are stored in a housing and the tape cassette formed in this manner is designed as a consumable article for a diagnostic device.

4. Tape unit according to one of the claims 1 to 3, **characterized in that** at least one of the spools (14, 16) has a coil core for receiving a tape coil that can be rotated by a motor or manually.

5. Tape unit according to one of the claims 1 to 4, **characterized in that** the inner spool (14, 16) located in the receiving area (26) is arranged eccentrically or concentrically relative to the surrounding outer spool (14, 16).

6. Tape unit according to one of the claims 1 to 5, **characterized in that** a measuring chamber for a measuring unit which optically scans the tape is kept clear in the receiving area and preferably within the spool (16) that is located therein.

7. Tape unit according to one of the claims 1 to 6, **characterized in that** the free section of tape (18) is guided on the front side over the outer spool (14) by means of a deflection guide (44, 46, 48).

8. Tape unit according to one of the claims 1 to 7, **characterized in that** the free section of tape (18) is guided over a first deflection element (44) arranged outside of the receiving area (26) and over a second guide element (46) arranged within the receiving area (26).

9. Tape unit according to claim 8, **characterized in that** the deflection elements are formed by deflection rollers (44, 46) having a preferably truncated conical shape.

10. Tape unit according to one of the claims 1 to 9, **characterized in that** the tape is guided at at least one deflection point over two preferably rounded deflection edges which run in different directions from one another.

11. Tape unit according to one of the claims 1 to 10, **characterized by** a transition structure (48) for the tape (12) arranged on a front side (52) of the spools (14, 16) which is preferably formed by a slide surface (50) or a seal.

12. Tape unit according to one of the claims 1 to 11, **characterized in that** the free section of tape (18) is passed on the front side over the outer spool (14) while twisting it longitudinally such that in a transition area the tape section (18) runs parallel to the front side (52) of the spool (14).

13. Tape unit according to one of the claims 1 to 12, **characterized in that** the tape (12) is provided in sections with analytical test fields (22) and that a test fluid and in particular a body fluid can be applied successively to the test fields (22) at an application site (20).

14. Tape unit according to one of the claims 1 to 11, **characterized in that** the lancing elements transported by means of the tape (12) can be provided at an application site (20) on the free section of tape (18).

15. Tape unit according to one of the claims 1 to 12, **characterized in that** at least the first spool (14) formed from unused tape is screened advantageously in the receiving area (26) from the environment.

16. Diagnostic system having a tape unit according to one of the previous claims wherein at least one spool (14) of the tape unit (10) can be rotated by a motor or manually.

## Revendications

1. Unité de bande diagnostique, en particulier cassette à bande, avec une bande (12) comportant des éléments de tests diagnostiques (22) et/ou des éléments piquants, laquelle bande peut être déroulée d'une première bobine (14) et enroulée sur une deuxième bobine (16), une partie de bande libre (18) située entre les bobines (14, 16) pouvant être mise en usage pour un utilisateur, **caractérisée en ce qu'**une des bobines (14, 16) entoure une zone de logement centrale (26) et **en ce que** l'autre bobine (14, 16) est située dans la zone de logement (26).

2. Unité de bande selon la revendication 1, **caractérisée en ce que** les deux bobines (14, 16) sont situées l'une dans l'autre à une même hauteur, les faces frontales de la bobine extérieure délimitant axialement la zone de logement (26).

3. Unité de bande selon la revendication 1 ou 2, **caractérisée en ce que** les deux bobines (14, 16) sont logées dans un boîtier et **en ce que** la cassette à bande ainsi formée se présente sous la forme d'un article consomptible pour un appareil diagnostique.

4. Unité de bande selon l'une des revendications 1 à 3, **caractérisée en ce qu'**au moins l'une des bobines (14, 16) présente un noyau d'enroulement à rotation motorisée ou manuelle pour loger un rouleau de bande.

5. Unité de bande selon l'une des revendications 1 à 4, **caractérisée en ce que** la bobine intérieure (14, 16) qui se trouve dans la zone de logement (26) est disposée excentriquement ou concentriquement par rapport à la bobine extérieure d'enceinte (14, 16).

6. Unité de bande selon l'une des revendications 1 à 5, **caractérisée en ce que,** dans la zone de logement, et de préférence à l'intérieur de la bobine (16) qui s'y trouve, une chambre de mesure est laissée libre pour une unité de mesure qui balaye la bande.

7. Unité de bande selon l'une des revendications 1 à 6, **caractérisée en ce que** la partie de bande libre (18) passe, du côté de la face frontale, sur la bande extérieure (14) par l'intermédiaire d'un guidage de renvoi (44, 46, 48).

8. Unité de bande selon l'une des revendications 1 à 7, **caractérisée en ce que** la partie de bande libre (18) passe par un premier élément de renvoi (44) situé à l'extérieur de la zone de logement (26) et par un deuxième élément de renvoi (46) situé à l'intérieur de la zone de logement (26).

9. Unité de bande selon la revendication 8, **caractérisée en ce que** les éléments de renvoi sont constitués par des rouleaux de renvoi (44, 46) de préférence tronconiques.

10. Unité de bande selon l'une des revendications 1 à 9, **caractérisée en ce que** la bande, en au moins un point de renvoi, passe par deux arêtes de renvoi orientées différemment de préférence arrondies.

11. Unité de bande selon l'une des revendications 1 à 10, **caractérisée par** une structure de transfert (48) pour la bande (12), située sur une face frontale (52) des bobines (14, 16) et constituée de préférence par une surface de glissement (50) ou un joint.

12. Unité de bande selon l'une des revendications 1 à 11, **caractérisée en ce que** la partie de bande libre (18) passe, du côté de la face frontale, sur la bobine extérieure (14) en s'enroulant longitudinalement de sorte que la partie de bande (18), dans une zone de transfert, est parallèle à la face frontale (52) de la bobine (14).

13. Unité de bande selon l'une des revendications 1 à 12, **caractérisée en ce que** la bande (12) est pourvue, par parties, de champs de tests analytiques (22) et **en ce que** les champs de tests (22), en un point d'application (20), peuvent être exposés successivement à un liquide de test, en particulier un liquide corporel.

14. Unité de bande selon l'une des revendications 1 à 11, **caractérisée en ce que** des éléments piquants transportés au moyen de la bande (12) peuvent être mis à disposition en un point d'application (20) sur la partie de bande libre (18).

15. Unité de bande selon l'une des revendications 1 à 12, **caractérisée en ce qu'**au moins la première bobine (14) constituée par une bande non utilisée est utilement protégée de l'environnement dans la zone de logement (26).

16. Système diagnostique avec une unité de bande selon l'une des revendications précédentes, au moins une bobine (14) de l'unité de bande (10) étant à rotation motorisée ou manuelle.
